# EUROPÄISCHE PATENTSCHRIFT

(11) **EP 4 262 904 B1**
(45) Veröffentlichungstag und Bekanntmachung des Hinweises auf die Patenterteilung: **20.11.2024**
(21) Anmeldenummer: 21839106.8
(22) Anmeldetag: 14.12.2021
(51) Int. Cl.: A61L 31/02, A61L 31/14, A61L 31/08

(54) **STENTS FÜR DIE ANWENDUNG IN DER INTERVENTIONELLEN BEHANDLUNG VON GEFÄSSERKRANKUNGEN UND DER GEFÄSSCHIRURGIE**
STENTS FOR USE IN THE INTERVENTIONAL TREATMENT OF VASCULAR DISORDERS AND VASCULAR SURGERY
ENDOPROTHÈSES DESTINÉES À ÊTRE UTILISÉES DANS LE TRAITEMENT INTERVENTIONNEL DES TROUBLES VASCULAIRES ET EN CHIRURGIE VASCULAIRE

(30) Priorität: 17.12.2020 DE 102020216158
(43) Veröffentlichungstag der Anmeldung: 25.10.2023
(73) Patentinhaber: Fraunhofer-Gesellschaft zur Förderung der angewandten Forschung e.V., 80686 München (DE)
(72) Erfinder: REDLICH, Christian, 01277 Dresden (DE); PÖHLE, Georg, 01277 Dresden (DE); QUADBECK, Peter, 01277 Dresden (DE)
(74) Vertreter: Pfenning, Meinig & Partner mbB
(86) Internationale Anmeldenummer: PCT/EP2021/085591
(87) Internationale Veröffentlichungsnummer: WO 2022/128979

(56) Entgegenhaltungen:
- EP-A2- 2 332 588
- US-A1- 2008 147 175

## Beschreibung

Die Erfindung betrifft Stents für die Anwendung in der interventionellen Behandlung von Gefäßerkrankungen und der Gefäßchirurgie.

Implantierbare Stents werden in der Kardiologie zur Therapie koronarer Gefäßverschlüsse und in der interventionellen Behandlung von Gefäßkrankheiten und der Gefäßchirurgie u. a. zur Behandlung von peripheren Stenosen, Aneurysmen, Aortendissektionen oder unfallbedingten Gefäßläsionen eingesetzt. Dadurch konnte z. B. im Fall der Koronarstents die Sterblichkeit aufgrund akuter Myokardinfarkte signifikant reduziert werden. Nach der Implantation muss die mechanische Integrität eines Stents bis zur Remodellierung des Gefäßes gewährleistet sein. Diese Dauer bemisst sich je nach Anwendungsfall, Art und Schwere der Läsion sowie dem Zustand des Patienten. Im Anschluss an diese funktionelle Lebensdauer ist es jedoch vorteilhaft, wenn der Stent wieder aus dem Gefäß verschwindet, um spät auftretende Komplikationen wie Restenosen oder Thrombosen zu vermeiden.

Bei Stents aus klassischen Materialien wie Stahl ist das nicht möglich. Es wurden daher bioresorbierbare Stents auf Basis von Magnesium oder Polymeren (z. B. Poly-L-Lactid) entwickelt und auf dem Markt eingeführt. Aufgrund der niedrigen Steifigkeiten und Festigkeiten dieser Werkstoffe sind jedoch verhältnismäßig groß dimensionierte Stentstreben (≥ 100 µm) im Vergleich zu handelsüblichen Stents aus medizinischem Edelstahl oder CoCr mit 60 - 80 µm Strebenweite und - dicke erforderlich. Allerdings zeigen aktuelle Publikationen, dass die bislang verfügbaren Polymer-Stents gegenüber herkömmlichen Stents keinen Vorteil bieten, sondern im Gegenteil eher eine erhöhte Sterblichkeit aufgrund höherer Thrombose-Raten verursachen. Als Ursachen für diese überraschenden Ergebnisse werden vor allem zu schnell auflösbare Scaffolds und zu hohe Strebendicken angesehen. Von den resorbierbaren Polymeren ist zudem bekannt, dass ihre Abbauprodukte zu unerwünschten Entzündungsreaktionen führen. Das Metall Magnesium degradiert darüber hinaus ungleichmäßig, was eine Vorhersage der zeitlichen Veränderung der Stenteigenschaften nach der Implantation erschwert.

Es besteht somit ein großer Bedarf an Stents aus einem resorbierbaren Material mit optimierten Festigkeits- und Degradationseigenschaften. Dies betrifft insbesondere Stents, die nach einer der Anwendung angepassten funktionellen Lebensdauer (Zeitraum mechanischer Integrität) schnell resorbiert werden sollen.

So ist aus US 2008 /147175 A eine bioresorbierbare endoluminale Prothese zum Einsetzen in ein Körperlumen mit einem Stent-Substrat aus einem ersten metallischen Material, das ein niedrigeres elektrisches Potential als eine Standard-Referenzelektrode aufweist. Das Stent-Substrat ist mit einem biologisch abbaubaren Polymer beschichtet, in dem ein zweites metallisches Material dispergiert ist, wobei das zweite metallische Material ein höheres elektrisches Potential als die Standard-Referenzelektrode aufweist. Nach der Implantation des Stents in das Körperlumen ist das zweite metallische Material in der Polymerbeschichtung in einer ausreichenden Konzentration vorhanden, um eine galvanische Korrosion des ersten metallischen Materials zu bewirken, so dass das Stentsubstrat mit der Zeit bioresorbiert wird.

Es ist daher Aufgabe der Erfindung, Möglichkeiten zur Verfügung zu stellen, mit denen die Eigenschaften von implantierten bioresorbierbaren Stents definiert und insbesondere der zeitliche Verlauf der Degradation und Resorption unter Berücksichtigung von mechanischer Integrität beeinflusst werden können.

Erfindungsgemäß wird diese Aufgabe mit einem Stent, der die Merkmale des Anspruchs 1 aufweist, gelöst. Vorteilhafte Weiterbildungen und Ausführungsformen können mit in abhängigen Ansprüchen bezeichneten Merkmalen realisiert werden.

Im Sinne der Erfindung wird ein Material als bioresorbierbar bezeichnet, wenn es im Körper abbaubar ist und die Abbauprodukte entweder direkt aus dem Körper ausgeschieden werden oder im Zuge regulärer Stoffwechselprozesse verwendet bzw. in vom Körper verwendbare Formen umgewandelt werden. Ein Implantat oder ein Teil eines Implantats, das aus einem bioresorbierbaren Material gebildet ist, verliert nach der Implantation mit der Zeit seine ursprüngliche Form. Während des Abbaus können die Konzentrationen der im Material enthaltenen Elemente im Körper über die Normalwerte hinausgehen. Nach dem vollständigen Abbau des Materials am Ort der Implantation gehen diese Konzentrationen wieder auf die Normalwerte zurück.

Die erfindungsgemäße Lösung besteht in einem Stent, der mit zwei bioresorbierbaren metallischen Werkstoffen gebildet ist.

Der erfindungsgemäße Stent ist mit einer rohrförmigen Stützstruktur, die aus miteinander verbundenen Streben, die aus einem ersten bioresorbierbaren metallischen Werkstoff gebildet sind, gebildet. Auf der Oberfläche der Streben ist eine Beschichtung, die mit einem zweiten metallischen bioresorbierbaren Werkstoff gebildet ist, ausgebildet. Dabei weist der zweite metallische Werkstoff eine unter physiologischen Bedingungen im implantierten Zustand kleinere Auflösungsrate bei der Bioresorption und ein positiveres Elektrodenpotential als der erste metallische Werkstoff auf. Sollten ein erster und ein zweiter metallischer Werkstoff ein negatives Elektrodenpotential gegenüber einer gängigen Bezugselektrode (z. B. Standardwasserstoff- oder Kalomelelektrode) aufweisen, ist der absolute Betrag des Elektrodenpotentials des zweiten metallischen Werkstoffs also kleiner als der absolute Betrag des Elektrodenpotentials des ersten metallischen Werkstoffs. Das Elektrodenpotential des zweiten metallischen Werkstoffs sollte unter physiologischen Bedingungen bevorzugt um +150 mV höher liegen als das Elektrodenpotential des ersten metallischen Werkstoffs, um einen möglichst großen galvanischen Effekt zu erzielen.

Die Summe des Volumens des zweiten metallischen Werkstoffs ist kleiner als das Volumen des ersten metallischen Werkstoffs, mit dem die Stentstreben gebildet sind.

Der erste metallische Werkstoff ist Wolfram, Molybdän oder eine Basislegierung eines dieser beiden Metalle. Dabei ist mindestens ein in einer Molybdän-Basislegierung enthaltenes Metall ausgewählt aus W, Re, Nb, Ta und Mn oder mindestens ein in einer Wolfram-Basislegierung enthaltenes Metall ausgewählt aus Mo, Re, Nb, Ta und Mn sein.

Eine Molybdän-Basislegierung besteht aus mindestens 50 at-% Mo und eine Wolfram-Basislegierung besteht aus mindestens 50 at-% Wolfram. Diese metallischen Werkstoffe weisen eine sehr hohe Festigkeit und Steifigkeit auf, wodurch sie die Bildung dünner Stentstreben erlauben. Die Werkstoffe zeichnen sich zudem durch einen gleichbleibenden Abtrag über die gesamte Oberfläche durch Korrosion bzw. Bioresorption aus. Da die Legierungselemente W, Ta und Nb in jedem Verhältnis mit Mo mischbar sind, kann eines oder mehrere dieser Elemente in einem beliebigen Anteil größer 0 at-% und kleiner 50 at-% Bestandteil einer Molybdän-Basislegierung sein. Gleiches gilt für die Legierungselemente Mo, Ta und Nb in einer Wolfram-Basislegierung. Zudem kann eine Mo-Basislegierung Rhenium mit mehr als 0 at.-% und maximal 42 at-% und Mangan mit mehr als 0 at-% und maximal 36 at-% enthalten. Eine W-Basislegierung kann Rhenium mit mehr als 0 at-% und maximal 37 at-% und Mangan mit mehr als 0 at-% und maximal 20 at-% enthalten.

Ein zweiter metallischer Werkstoff ist reines Rhenium, oder eine Legierung von Rhenium mit Molybdän oder Wolfram. Als zweiter metallischer Werkstoff kann eine Basislegierung von Rhenium Molybdän oder Wolfram eingesetzt werden, die mehr als 0 at-% und maximal 14 at-% Molybdän enthalten kann, oder eine Basislegierung von Rhenium mit Wolfram eingesetzt werden, die mehr als 0 at-% und maximal 20 at-% Wolfram enthält. In einer Molybdänoder Wolframbasislegierung enthaltene Metall ist Rhenium. Es kann auch eine Legierung von Molybdän und Rhenium als zweiter metallischer Werkstoff eingesetzt werden, die mehr als 0 at-% und maximal 42 at-% Rhenium enthält, bzw. eine Legierung von Wolfram und Rhenium, wobei diese Legierung mehr als 0 at-% und maximal 37 at-% Rhenium enthält. Ist der erste metallische Werkstoff eine Legierung mit Rhenium, enthält der zweite metallische Werkstoff einen größeren Anteil an Rhenium und hat ein positiveres Elektrodenpotential als der erste metallische Werkstoff. Ist der erste metallische Werkstoff Wolfram oder eine Wolfram-Basislegierung ohne Rhenium, kann der zweite metallische Werkstoff auch reines Molybdän sein.

Der zweite metallische Werkstoff kann mittels verschiedener Beschichtungsmethoden auf dem ersten metallischen Werkstoff aufgebracht werden. Beispiele sind Methoden der chemischen Gasphasenabscheidung (chemical vapour deposition, PVD), wie z. B. die Atomlagenabscheidung (atomic layer deposition), oder der physikalischen Gasphasenabscheidung (physical vapor deposition, CVD), wie z. B. das Magnetron-Sputtern oder das lonenstrahl-Sputtern.

Der erste metallische Werkstoff soll vollständig und vollflächig vom zweiten metallischen Werkstoff bedeckt sein, um während des Funktionszeitraums eine schnelle Korrosion des ersten metallischen Werkstoffs zu verhindern und die mechanische Integrität des Stents zu gewährleisten.

Die Dicke der Beschichtung, die mit dem zweiten metallischen Werkstoff gebildet worden ist, sollte im Bereich 1 nm bis 1000 nm, bevorzugt 1 nm - 50 nm gewählt werden. Bevorzugt erfolgt die Beschichtung mit dem zweiten metallischen Werkstoff so, dass die Schichtdicke über die Stentoberfläche ungleichmäßig ist. Die Dicke der Beschichtung sollte unter Berücksichtigung der Auflösungsrate der metallischen Werkstoffe infolge Bioresorption und elektrochemischer Korrosion sowie der für die Wiederherstellung der Gefäßwand erforderlichen Zeit ausgebildet werden. Ein Teil der Beschichtung kann zum Zeitpunkt, an dem die jeweilige Gefäßwand einen ausreichend gesunden Zustand erreicht hat, bereits abgebaut sein, solange die mechanische Integrität des Stents dadurch nicht gefährdet ist. Dabei können beispielsweise der Gesundheitszustand und das Alter eines Patienten vor der Operation, die Art des den implantierten Stent aufnehmenden Gefäßes sowie die Art und Schwere der Läsion für die erforderliche Dicke der Beschichtung mit dem zweiten Werkstoff berücksichtigt werden.

Die Beschichtung kann entweder auf die elektropolierte Oberfläche des ersten metallischen Werkstoffs oder nach Aufprägung einer gesonderten Oberflächenstruktur auf die jeweiligen Oberflächenbereiche des ersten metallischen Werkstoffs aufgebracht werden. Dies betrifft vorrangig die Oberflächenbereiche der Streben des Stents, die in Gefäßwandrichtung angeordnet bzw. ausgerichtet sind. Diese können mit einer Oberflächenstrukturierung, die mit Erhebungen und Vertiefungen gebildet ist, versehen sein.

Die Oberfläche der Streben kann mit der Oberflächenstrukturierung um einem Faktor 1,1 bis 10 im Vergleich zu einer elektropolierten Oberfläche der Streben vergrößert worden sein.

Die Oberflächenstrukturierung kann vorteilhaft periodisch und/oder mit Rillen, Senken, Tälern als Vertiefungen und/oder Erhebungen mit Ringen und/oder Bergen ausgebildet worden sein.

Die Oberflächenstrukturierung kann durch einen lokal definierten Werkstoffabtrag, bevorzugt im Bereich der Oberfläche der Streben der Stents, die der Gefäßwand zugewandt sind, unter Einwirkung von Laserstrahlung oder fotolithografischer Techniken erreicht werden. Sie kann auch durch eine allseitige definierte Ätzung einer Stentstruktur oder einer als Halbzeug verwendeten Röhre aus dem ersten metallischen Werkstoff, z. B. mit Wasserstoffperoxid, ausgebildet werden.

Durch den Aufbau des Stents aus zwei bioresorbierbaren metallischen Werkstoffen können ein für die interventionelle Kardiologie oder Gefäßchirurgie günstiges Resorptionsverhalten unter physiologischen Bedingungen und die Zeit bis zu einer vollständigen Auflösung der metallischen Werkstoffe eingestellt werden.

Die Auflösung des erfindungsgemäßen implantierten Stents im Gefäß ist durch drei zeitliche Abschnitte mit unterschiedlich hohen Auflösungsraten gekennzeichnet. Die Dauer der Zeitabschnitte lässt sich insbesondere über die Dicke der Beschichtung regulieren, so dass sich das Auflösungsverhalten des Stents in einfacher Weise an den jeweiligen Anwendungsfall anpassen lässt.

Im ersten Zeitabschnitt ist die Auflösungsrate gering, weil nur der langsam abbaubare zweite metallische Werkstoff exponiert ist und resorbiert wird. In diesem Zeitabschnitt, der der funktionellen Lebensdauer entsprechen soll, sind die mechanischen Eigenschaften des Stents daher konstant, da sie vordergründig vom ersten metallischen Werkstoff bestimmt werden, der in diesem Zeitraum vor Degradation geschützt wird.

Der zweite Zeitabschnitt beginnt, wenn der erste metallische Werkstoff durch den Abbau des zweiten metallischen Werkstoffs partiell freigelegt wurde. Die partielle Freilegung kann durch eine ungleichmäßige Dicke der Beschichtung auf der Oberfläche der Streben mit dem zweiten metallischen Werkstoff gefördert werden. Bei gleichzeitiger Exposition beider metallischer Werkstoffe kommt es in diesem Zeitabschnitt aufgrund der unterschiedlichen Elektrodenpotentiale zu galvanischer Korrosion und der erste metallische Werkstoff wird in vom zweiten metallischen Werkstoff freigelegten Oberflächenbereichen lokal beschleunigt abgebaut, während der zweite metallische Werkstoff lokal vor weiterer Korrosion geschützt wird. Zusätzlich wird der Abbau des ersten metallischen Werkstoffs durch die Oberflächenrauheit/-strukturierung beschleunigt, unter Berücksichtigung der über eine gesamte exponierte Fläche besonders gleichmäßig stattfindenden Degradation von Molybdän und Wolfram.

Im dritten Zeitabschnitt ist die Auflösungsrate geringer als im zweiten Zeitabschnitt, da der Einfluss der galvanischen Korrosion durch fortgeschrittene Auflösung bzw. die Fragmentierung der Beschichtung aus dem zweiten metallischen Werkstoff schwächer wird. Die Oberflächenrauheit/-strukturierung wirkt sich jedoch wegen der größeren Oberfläche weiterhin positiv auf die Auflösungsgeschwindigkeit aus. Die Auflösungsrate liegt wesentlich höher als im ersten Zeitabschnitt, da die Auflösungsrate des ersten metallischen Werkstoffs grundsätzlich höher ist als die des zweiten metallischen Werkstoffs.

Nachfolgend soll die Erfindung beispielhaft näher erläutert werden.

Dabei zeigt:

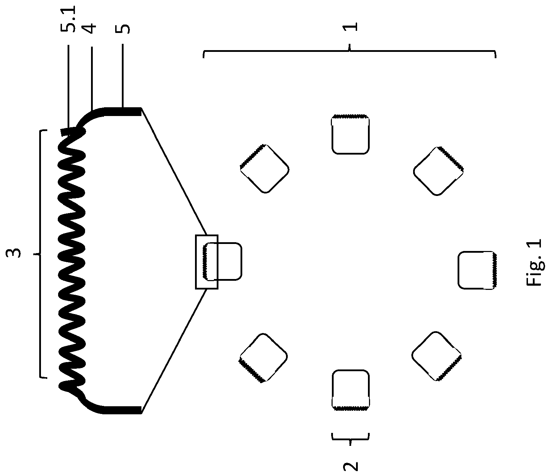

Figur 1 eine geschnittene Draufsicht auf ein Beispiel eines erfindungsgemäßen Stents mit einer vergrößerten Teildarstellung.

In Figur 1 ist eine geschnittene Draufsicht in einer Ebene, die senkrecht zur mittleren Längsachse des Stents 1 ausgerichtet ist, gezeigt. Der Stent 1 ist mit Streben 2 gebildet, die punktuell miteinander verbunden sind (nicht gezeigt). Zwischen den Streben 2 sind Freiräume vorhanden, wie es auch bei herkömmlichen Stents 1 der Fall ist.

Wie man insbesondere der oben gezeigten vergrößerten Teildarstellung entnehmen kann, ist der Oberflächenbereich der Streben 2, der der Gefäßwand zugewandt ist, mit einer Oberflächenstrukturierung 3 versehen worden, die mit Rillen als Vertiefungen und Ringen als Erhebungen gebildet worden ist. Die Rillen und Ringe sind bei diesem Beispiel periodisch wiederholend ausgebildet worden.

Auf den Streben 2, die aus dem ersten metallischen Werkstoff 4 bestehen, ist eine Beschichtung 5.1, die mit dem zweiten metallischen Werkstoff 5 gebildet ist, ausgebildet.

Die Dimensionierung aller Elemente des Stents 1 kann entsprechend den Angaben im allgemeinen Teil der Beschreibung gewählt werden.

### Ausführungsbeispiel 1:

Dieses Ausführungsbeispiel beschreibt einen kardiovaskulären Stent 1. Figur 1 zeigt eine schematische Schnittdarstellung eines Beispiels eines Stents 1 bestehend aus mehreren Streben 2, die in nicht gezeigter Art und Weise punktuell miteinander verbunden sind, wobei die Verbindungspunkte in Ebenen angeordnet sind, die von der Ebene des gezeigten Schnittes einen Abstand haben. Der erste metallische Werkstoff 4, aus dem die Streben 2 bestehen, ist reines Molybdän. Diese Struktur wird erzeugt, indem durch gängige Ziehverfahren ein Molybdän-Röhrchen mit 3 mm Durchmesser und einer Wandstärke von 50 µm erhalten wird. Die Wandstärke ergibt dabei die Strebendicke (radiale Ausdehnung) der späteren Stentstruktur. Aus diesem Röhrchen wird anschließend mittels Laserschneidverfahren eine Stentstruktur mit einer Länge von 30 mm hergestellt, wobei die Strebenweite (tangentiale Ausdehnung) der punktuell miteinander verbundenen Streben 250 µm beträgt.

Anschließend wird die mit den Streben 2 gebildete Struktur gereinigt und mittels Elektropoliturverfahren entgratet. Mithilfe eines Ultrakurzpulslasers wird auf der Oberfläche der Streben 2, die der Gefäßwand zugewandt ist, eine Oberflächenstrukturierung 3 mit Rillen als Vertiefungen entlang der Länge der Streben 2 mit einer mittleren Strukturhöhe von 5 µm erzeugt und so die Gesamtoberfläche um einen Faktor von 2,5 erhöht. Anschließend wird mittels des Verfahrens der Atomlagenabscheidung eine defektfreie Beschichtung 5.1 aus reinem Rhenium als zweiten metallischen Werkstoff 5 mit einer mittleren Schichtdicke von 20 nm aufgebracht. Die Beschichtung 5.1 umschließt den Kern der Streben 2, die aus dem ersten metallischen Werkstoff 4 bestehen, vollständig. Rhenium hat eine Auflösungsrate von 50 nm pro Jahr. Die Dicke der Beschichtung 5.1 ist so gewählt, dass sie für die notwendige Funktionsdauer des Stents 1 von 4 Monaten dessen mechanische Integrität gewährleistet, indem sie das Molybdän vor Korrosion schützt. Die Dicke der Beschichtung 5.1 variiert leicht, insbesondere im oberflächenstrukturierten Bereich 3 der Streben 2.

Nach Ende der Funktionsdauer, in der die Gefäßwand eines Patienten vollständig geheilt ist, hat sich das Rhenium lokal an mehreren Stellen der Streben 2 aufgelöst, so dass das darunterliegende Molybdän freiliegt. Dies geschieht bevorzugt im oberflächenstrukturierten Bereich 3, da hier unterschiedlich dicke Bereiche der Beschichtung 5.1 gebildet wurden. Durch Bildung lokaler galvanischer Elemente wird die Korrosion des freilegenden Molybdäns beschleunigt, während das umliegende Rhenium vor weiterer Korrosion geschützt ist. Durch die lokal begrenzte Wirkung der galvanischen Elemente bilden sich mit der Zeit weitere galvanische Lokalelemente über die gesamte Oberfläche der Stentstruktur.

Die erhöhte Oberfläche im strukturierten Bereich 3 sorgt für eine zusätzliche Beschleunigung der Auflösung und Resorption des Stents 1. Die lokale Korrosion schwächt außerdem die Integrität des Stents 1. Dies führt schließlich zu einem Bruch der Stentstruktur, was wiederum zu einer Vergrößerung der Oberfläche und damit einer verstärkten Auflösung des Molybdäns führt. Ohne Einfluss von galvanischer Korrosion liegt die Auflösungsrate von Molybdän bei 25 µm pro Jahr. Die Dauer der vollständigen Degradation dieses Stents 1 liegt bei etwa einem Jahr.

### Ausführungsbeispiel 2:

Das Ausführungsbeispiel beschreibt einen peripheren Stent für Beinarterien. Durch gängige Ziehverfahren wird ein Röhrchen aus einer Molybdän-Legierung mit 25 at.-% Wolfram mit 5 mm Durchmesser und einer Wandstärke von 70 µm hergestellt. Die Wandstärke ergibt dabei die Strebendicke (radiale Ausdehnung) der späteren Stentstruktur. Aus diesem Röhrchen wird anschließend mittels Laserschneidverfahren eine Stentstruktur mit einer Länge von 50 mm hergestellt, wobei die Strebenweite (tangentiale Ausdehnung) der punktuell miteinander verbundenen Streben 2 60 µm beträgt. Anschließend wird die Oberfläche der Streben 2 gereinigt und mittels Elektropoliturverfahren entgratet. Daraufhin wird die Gesamtoberfläche mittels Ätzung mit Wasserstoffperoxid durch Aufrauen um den Faktor 1,5 erhöht. Anschließend wird mittels des Verfahrens der Atomlagenabscheidung eine defektfreie Beschichtung 5.1 aus reinem Rhenium 5 mit einer mittleren Schichtdicke von 5 nm aufgebracht. Die Beschichtung 5.1 umschließt den Kern der Streben 2 vollständig. Rhenium als zweiter metallischer Werkstoff 5 hat eine jährliche Auflösungsrate von 50 nm. Die Dicke der Beschichtung 5.1 ist so gewählt, dass die mechanische Integrität des Stents für eine Dauer von mindestens einem Monat gewährleistet ist, indem die Beschichtung 5.1 die Molybdän-Wolfram-Legierung als erster metallischer Werkstoff 4 vor Korrosion schützt. Nach Ende der Funktionsdauer, innerhalb derer die Gefäßwand des Patienten vollständig geheilt ist, bilden sich durch lokale Freilegung des Rheniums infolge der Auflösung lokale galvanische Elemente mit der darunterliegenden Molybdän-Wolfram-Legierung. Die Korrosion der Molybdän-Wolfram-Legierung wird beschleunigt, während das umliegende Rhenium vor weiterer Korrosion geschützt wird. Durch die lokal begrenzte Wirkung der galvanischen Elemente bilden sich dennoch mit der Zeit weitere Lokalelemente über die gesamte Oberfläche der Streben 2 des Stents 1. Die lokale Korrosion schwächt die Integrität des Stents 1. Dies führt schließlich zu einem Bruch der Stentstruktur, was wiederum zu einer Vergrößerung der Oberfläche und damit einer verstärkten Auflösung der Molybdän-Wolfram-Legierung führt. Ohne Einfluss von galvanischer Korrosion liegt die Auflösungsrate der Molybdän-Wolfram-Legierung bei 35 µm pro Jahr. Die Dauer der vollständigen Degradation dieses Stents 1 liegt bei etwa einem Jahr.

### Ausführungsbeispiel 3:

Dieses Ausführungsbeispiel beschreibt einen Flow-Diverter-Stent 1 zur Behandlung von Aneurysmen. Figur 1 zeigt eine schematische Schnittdarstellung eines Beispiels eines Stents 1 bestehend aus mehreren Streben 2, die in nicht gezeigter Art und Weise punktuell miteinander verbunden sind, wobei die Verbindungspunkte in Ebenen angeordnet sind, die von der Ebene des gezeigten Schnittes einen Abstand haben. Der erste metallische Werkstoff 4, aus dem die Streben 2 bestehen, ist reines Wolfram. Diese Struktur wird erzeugt, indem durch gängige Ziehverfahren ein Wolfram-Röhrchen mit 3 mm Durchmesser und einer Wandstärke von 50 µm erhalten wird. Die Wandstärke ergibt dabei die Strebendicke (radiale Ausdehnung) der späteren Stentstruktur. Aus diesem Röhrchen wird anschließend mittels Laserschneidverfahren eine Stentstruktur mit einer Länge von 30 mm hergestellt, wobei die Strebenweite (tangentiale Ausdehnung) der punktuell miteinander verbundenen Streben 2 50 µm beträgt.

Anschließend wird die mit den Streben 2 gebildete Struktur gereinigt und mittels Elektropoliturverfahren entgratet. Mithilfe eines fotolithografischen Verfahrens in Verbindung mit einer Wasserstoffperoxidätzung wird auf der Oberfläche der Streben 2, die der Gefäßwand zugewandt ist, eine Oberflächenstrukturierung 3 mit definierten periodischen Vertiefungen entlang der Länge der Streben 2 mit einer mittleren Strukturhöhe von 2 µm erzeugt und so die Gesamtoberfläche um einen Faktor von 1,5 erhöht. Anschließend wird mittels des Verfahrens des Magnetronsputterns mit rotierendem Substrat eine Beschichtung 5.1 aus einem zweiten metallischen Werkstoff 5, einer Rheniumlegierung mit 15 at-% Wolfram, mit einer mittleren Schichtdicke von 600 nm aufgebracht. Die Beschichtung 5.1 umschließt den Kern der Streben 2, die aus dem ersten metallischen Werkstoff 4 bestehen, vollständig. Die Rhenium-Wolframlegierung hat eine Auflösungsrate von 3000 nm pro Jahr. Die Dicke der Beschichtung 5.1 ist so gewählt, dass sie für die notwendige Funktionsdauer des Stents 1 von 2 Monaten dessen mechanische Integrität gewährleistet, indem sie das Wolfram vor Korrosion schützt. Die Dicke der Beschichtung 5.1 variiert, insbesondere durch die Herstellung mittels Magnetronsputtern und im oberflächenstrukturierten Bereich 3 der Streben 2.

Nach Ende der Funktionsdauer, in der die Gefäßwand geheilt und das Aneurysma eines Patienten vollständig okkludiert ist, hat sich die Rhenium-Wolfram-Legierung lokal an mehreren Stellen der Streben 2 aufgelöst, so dass das darunterliegende Wolfram freiliegt. Dies geschieht bevorzugt im oberflächenstrukturierten Bereich 3, da hier unterschiedlich dicke Bereiche der Beschichtung 5.1 gebildet wurden. Durch Bildung lokaler galvanischer Elemente wird die Korrosion des freilegenden Wolframs beschleunigt, während die umliegende Rhenium-Wolfram-Legierung vor weiterer Korrosion geschützt ist. Durch die lokal begrenzte Wirkung der galvanischen Elemente bilden sich dennoch mit der Zeit weitere galvanische Lokalelemente über die gesamte Oberfläche der Stentstruktur.

Die erhöhte Oberfläche im strukturierten Bereich 3 sorgt für eine zusätzliche Beschleunigung der Auflösung und Resorption des Stents 1. Die lokale Korrosion schwächt außerdem die Integrität des Stents 1. Dies führt schließlich zu einem Bruch der Stentstruktur, was wiederum zu einer Vergrößerung der Oberfläche und damit einer verstärkten Auflösung des Wolframs führt. Ohne Einfluss von galvanischer Korrosion liegt die Auflösungsrate von Wolfram bei 40 µm pro Jahr. Die Dauer der vollständigen Degradation dieses Stents 1 liegt bei etwa 6 Monaten.

## Patentansprüche

1. Stent für die Anwendung in der interventionellen Behandlung von Gefäßerkrankungen und der Gefäßchirurgie, bei dem eine rohrförmige Stützstruktur, die mit punktuell miteinander verbundenen Stegen (2), die aus einem ersten bioresorbierbaren metallischen Werkstoff (4) gebildet sind, gebildet ist und
auf der Oberfläche der Stege (2) eine vollständig bedeckende Beschichtung (5.1), die mit einem zweiten bioresorbierbaren metallischen Werkstoff (5) gebildet ist, ausgebildet ist, wobei der zweite metallische Werkstoff (5) eine unter physiologischen Bedingungen im implantierten Zustand kleinere Auflösungsrate bei der Bioresorption und ein positiveres Elektrodenpotential gegenüber dem ersten metallischen Werkstoff (4) aufweist,
**dadurch gekennzeichnet, dass** der erste metallische Werkstoff (4) Wolfram, Molybdän oder eine Basislegierung eines dieser beiden Metalle ist, wobei mindestens ein in einer Molybdän-Basislegierung enthaltenes Metall ausgewählt ist aus W, Re, Nb, Ta und Mn oder mindestens ein in einer Wolfram-Basislegierung enthaltenes Metall ausgewählt ist aus Mo, Re, Nb, Ta und Mn, und
dass der zweite metallische Werkstoff (5) aus Rhenium oder einer Basislegierung aus Rhenium, Molybdän oder Wolfram gebildet ist, wobei mindestens ein in einer Rhenium-Basislegierung enthaltenes Metall ausgewählt ist aus W und Mo oder das in einer Molybdän- oder Wolframbasislegierung enthaltene Metall Re ist.

2. Stent nach Anspruch 1, **dadurch gekennzeichnet, dass** die Oberfläche der Stege (2), die aus dem ersten bioresorbierbaren metallischen Werkstoff (4) gebildet sind, teilweise oder vollständig mit einer Oberflächenstrukturierung (3), die mit Erhebungen und Vertiefungen gebildet ist, versehen sind.

3. Stent nach dem vorhergehenden Anspruch, **dadurch gekennzeichnet, dass** die Oberfläche der Stege (2), die aus dem ersten bioresorbierbaren metallischen Werkstoff (4) gebildet sind, mit der Oberflächenstrukturierung (3) um einem Faktor 1,1 bis 10 im Vergleich zu einer elektropolierten Oberfläche der Stege (2) vergrößert ist.

4. Stent nach einem der zwei vorhergehenden Ansprüche, **dadurch gekennzeichnet, dass** die Oberflächenstrukturierung (3) periodisch und/oder mit Rillen, Senken, Tälern und/oder Erhebungen mit Ringen und/oder Bergen ausgebildet ist.

5. Stent nach einem der vorhergehenden Ansprüche, **dadurch gekennzeichnet, dass** der erste metallische Werkstoff (4) aus einer Molybdänbasis-Legierung, in der mindestens 50 at-% Mo oder einer WolframBasislegierung, in der mindestens 50 at-% Wolfram enthalten sind, gebildet ist.

6. Stent nach dem vorhergehenden Anspruch, **dadurch gekennzeichnet, dass** eine Molybdän-Legierung mit mindestens einem Legierungselement, das ausgewählt ist aus W, Ta, Nb, Re und Mn, gebildet ist und das/die Legierungselement(e) W, Ta und/oder Nb mit einem Anteil größer 0 at-% bis kleiner 50 at-%, und/oder das Legierungselement Re mit einem Anteil größer 0 at-% bis 42 at-% und/oder das Legierungselement Mn mit einem Anteil größer 0 at-% bis 36 at-% enthält.

7. Stent nach Anspruch 5, **dadurch gekennzeichnet, dass** eine Wolfram-Legierung mit mindestens einem Legierungselement, das ausgewählt ist aus Mo, Ta, Nb, Re und Mn, gebildet ist und das/die Legierungselement(e) Mo, Ta und/oder Nb mit einem Anteil größer 0 at-% bis kleiner 50 at-%, und/oder das Legierungselement Re mit einem Anteil größer 0 at-% bis 37 at-% und/oder das Legierungselement Mangan mit einem Anteil größer 0 at-% bis 20 at-% enthält.

8. Stent nach einem der vorhergehenden Ansprüche, **dadurch gekennzeichnet, dass** eine Rhenium-Basislegierung mit einem Anteil größer 0 at-% bis 14 at-% an Mo oder mit einem Anteil größer 0 at-% bis 20 at-% an W gebildet ist.

9. Stent nach einem der vorhergehenden Ansprüche, **dadurch gekennzeichnet, dass** eine Molybdän-Basislegierung mit einem Re-Anteil größer 0 at-% bis 42 at-% gebildet ist, wobei der Anteil an Re größer ist als der Re-Anteil in dem ersten metallischen Werkstoff, wenn dieser erste Werkstoff (4) eine MoRe-Legierung ist.

10. Stent nach einem der vorhergehenden Ansprüche, **dadurch gekennzeichnet, dass** eine Wolfram-Basislegierung mit einem Re-Anteil größer 0 at-% bis 37 at-% beinhaltet, wobei der Anteil an Re größer ist als der Re-Anteil in dem ersten metallischen Werkstoff, wenn dieser erste Werkstoff (4) eine WRe-Legierung ist.

11. Stent nach einem der vorhergehenden Ansprüche, **dadurch gekennzeichnet, dass** die Summe des Volumens des zweiten metallischen Werkstoffs (5) kleiner als das Volumen des ersten metallischen Werkstoffs (4), mit dem die Stentstege (2) gebildet sind, ist.

12. Stent nach einem der vorhergehenden Ansprüche, **dadurch gekennzeichnet, dass** die Beschichtung (5.1) mit einer Schichtdicke im Bereich 1 nm bis 1000 nm, bevorzugt mit 1 nm bis 50 nm unter Berücksichtigung der Auflösungsrate und der für die Wiederherstellung der Gefäßwand erforderlichen Zeit ausgebildet ist.

13. Stent nach einem der vorhergehenden Ansprüche, **dadurch gekennzeichnet, dass** die Schichtdicke der Beschichtung (5.1) auf der Oberfläche der Stege (2) variiert ist.

## Claims

1. A stent for use in the interventional treatment of vascular diseases and vascular surgery, in which a tubular support structure, which is formed of struts (2) that are connected to one another at selective points and made of a first bioresorbable metallic material (4), is formed, and
a completely covering coating (5.1), which is produced with a second bioresorbable metallic material (5), is created on the surface of the struts (2), wherein
the second metallic material (5) having a lower dissolution rate under physiological conditions when implanted during bioresorption and a more positive electrode potential compared to the first metallic material (4),
**characterized in that** the first metallic material (4) is tungsten, molybdenum, or a base alloy of one of these two metals, at least one metal contained in a molybdenum base alloy being selected from W, Re, Nb, Ta and Mn, or at least one metal contained in the tungsten base alloy being selected from Mo, Re, Nb, Ta and Mn, and
**in that** the second metallic material (5) is made of rhenium or a base alloy of rhenium, molybdenum or tungsten, at least one metal contained in the rhenium base alloy being selected from W and Mo or the metal contained in a molybdenum or tungsten base alloy being Re.

2. The stent according to claim 1, **characterized in that** the surface of the struts (2), which are made of the first bioresorbable metallic material (4), is partially or completely provided with a surface structuring (3), which is produced with elevations and depressions.

3. The stent according to the preceding claim, **characterized in that** the surface of the struts (2), which are made of the first bioresorbable metallic material (4), is increased with a surface structuring (3) by a factor of 1.1 to 10 compared to an electropolished surface of the struts (2).

4. The stent according to one of the two preceding claims, **characterized in that** the surface structuring (3) is created periodically and/or using grooves, troughs, valleys and/or elevations, using rings and/or peaks.

5. The stent according to any one of the preceding claims, **characterized in that** the first metallic material (4) is made of a molybdenum base alloy which contains at least 50 at% Mo or a tungsten base alloy which contains at least 50 at% tungsten.

6. The stent according to the preceding claim, **characterized in that** a molybdenum alloy is produced with at least one alloying element which is selected from W, Ta, Nb, Re and Mn and comprises the alloying element(s) W, Ta and/or Nb in a content of greater than 0 at% to less than 50 at% and/or the alloying element Re in a content of greater than 0 at% to 42 at% and/or the alloying element Mn in a content of greater than 0 at% to 36 at%.

7. The stent according to claim 5, **characterized in that** a tungsten alloy is produced with at least one alloying element which is selected from Mo, Ta, Nb, Re and Mn and comprises the alloying element(s) Mo, Ta and/or Nb in a content of greater than 0 at% to less than 50 at% and/or the alloying element Re in a content of greater than 0 at% to 37 at% and/or the alloying element manganese in a content of greater than 0 at% to 20 at%.

8. The stent according to the preceding claims, **characterized in that** a rhenium base alloy having a content of greater than 0 at% to 14 at% of Mo or a content of greater than 0 at% to 20 at% of W is produced.

9. The stent according to any one of the preceding claims, **characterized in that** a molybdenum base alloy having a Re content of greater than 0 at% to 42 at% is produced, the content of Re being greater than the Re content in the first metallic material when this first material (4) is a MoRe alloy.

10. The stent according to any one of the preceding claims, **characterized in that** a tungsten base alloy having a Re content of greater than 0 at% to 37 at% is produced, the content of Re being greater than the Re content in the first metallic material when this first material (4) is a WRe alloy.

11. The stent according to any one of the preceding claims, **characterized in that** the sum of the volume of the second metallic material (5) is less than the volume of the first metallic material (4) that is used to produce the stent struts (2).

12. The stent according to any one of the preceding claims, **characterized in that** the coating (5.1) is created with a layer thickness in the range of 1 nm to 1000 nm, and preferably 1 nm to 50 nm, taking into consideration the dissolution rate and the time required for the restoration of the vessel wall.

13. The stent according to any one of the preceding claims, **characterized in that** the layer thickness of the coating (5.1) on the surface of the struts (2) varies.

## Revendications

1. Endoprothèse vasculaire pour une utilisation dans le traitement interventionnel des maladies vasculaires et dans la chirurgie vasculaire, pour lequel une structure de support tubulaire est formée avec des entretoises (2) reliées ponctuellement entre elles et réalisées à partir d'un premier matériau métallique biorésorbable (4) et
sur la surface des entretoises (2) est formé un revêtement (5.1) entièrement couvrant réalisé à partir d'un deuxième matériau métallique biorésorbable (5), dans lequel
le deuxième matériau métallique (5) présente un taux de dissolution plus faible en biorésorption dans des conditions physiologiques à l'état implanté et un potentiel d'électrode plus positif par rapport au premier matériau métallique (4),
**caractérisée en ce que** le premier matériau métallique (4) est le tungstène, le molybdène ou un alliage de base de l'un de ces deux métaux, dans lequel au moins un métal contenu dans un alliage de base de molybdène est choisi parmi W, Re, Nb, Ta et Mn ou
au moins un métal contenu dans un alliage de base de tungstène est choisi parmi Mo, Re, Nb, Ta et Mn, et
**en ce que** le deuxième matériau métallique (5) est constitué de rhénium ou d'un alliage de base de rhénium, de molybdène ou de tungstène, dans lequel au moins un métal contenu dans un alliage de base de rhénium est choisi parmi W et Mo, ou le métal contenu dans un alliage de base de molybdène ou de tungstène est Re.

2. Endoprothèse vasculaire selon la revendication 1, **caractérisée en ce que** la surface des entretoises (2) réalisées à partir du premier matériau métallique biorésorbable (4) est partiellement ou totalement munie d'une structuration de surface (3) formée de bosses et de creux.

3. Endoprothèse vasculaire selon la revendication précédente, **caractérisée en ce que**, avec la structuration de surface (3), la surface des entretoises (2) réalisées à partir du premier matériau métallique biorésorbable (4) est augmentée d'un facteur compris entre 1,1 et 10 par rapport à une surface électro-polie des entretoises (2).

4. Endoprothèse vasculaire selon l'une quelconque des deux revendications précédentes, **caractérisée en ce que** la structuration de surface (3) est réalisée de manière périodique et/ou avec des rainures, des creux, des vallées et/ou des surélévations avec des anneaux et/ou des pics.

5. Endoprothèse vasculaire selon l'une quelconque des revendications précédentes, **caractérisée en ce que** le premier matériau métallique (4) est constitué d'un alliage de base de molybdène contenant au moins 50 % at de Mo, ou d'un alliage de base de tungstène contenant au moins 50 % at de tungstène.

6. Endoprothèse vasculaire selon la revendication précédente, **caractérisée en ce qu'**un alliage de molybdène est réalisé avec au moins un élément d'alliage choisi parmi W, Ta, Nb, Re et Mn, et contient le ou les élément(s) d'alliage W, Ta et/ou Nb en une proportion comprise entre plus de 0 % at et moins de 50 % at et/ou contient l'élément d'alliage Re en une proportion comprise entre plus de 0 % at et 42 % at et/ou contient l'élément d'alliage Mn en une proportion comprise entre plus de 0 % at et 36 % at.

7. Endoprothèse vasculaire selon la revendication 5, **caractérisée en ce qu'**un alliage de tungstène est réalisé avec au moins un élément d'alliage choisi parmi Mo, Ta, Nb, Re et Mn, et contient le ou les élément(s) d'alliage Mo, Ta et/ou Nb en une proportion comprise entre plus de 0 % at et moins de 50 % at et/ou contient l'élément d'alliage Re en une proportion comprise entre plus de 0 % at et 37 % at et/ou contient l'élément d'alliage manganèse en une proportion comprise entre plus de 0 % at et 20 % at.

8. Endoprothèse vasculaire selon l'une quelconque des revendications précédentes, **caractérisée en ce qu'**un alliage de base de rhénium est réalisé avec une proportion de Mo comprise entre plus de 0 % at et 14 % at ou avec une proportion de W comprise entre plus de 0 % at et 20 % at.

9. Endoprothèse vasculaire selon l'une quelconque des revendications précédentes, **caractérisée en ce qu'**un alliage de base de molybdène est réalisé avec une proportion de Re comprise entre plus de 0 % at et 42 % at, dans lequel la proportion de Re est supérieure à la proportion de Re dans le premier matériau métallique si ledit premier matériau (4) est un alliage MoRe.

10. Endoprothèse vasculaire selon l'une quelconque des revendications précédentes, **caractérisée en ce qu'**un alliage de base de tungstène contient une proportion de Re comprise entre plus de 0 % at et 37 % at, dans lequel la proportion de Re est supérieure à la proportion de Re dans le premier matériau métallique si ledit premier matériau (4) est un alliage WRe.

11. Endoprothèse vasculaire selon l'une quelconque des revendications précédentes, **caractérisée en ce que** le total du volume du deuxième matériau métallique (5) est inférieur au volume du premier matériau métallique (4) avec lequel les entretoises d'endoprothèse vasculaire (2) sont réalisées.

12. Endoprothèse vasculaire selon l'une quelconque des revendications précédentes, **caractérisée en ce que** le revêtement (5.1) est formé avec une épaisseur de couche située dans la plage comprise entre 1 nm et 1000 nm, de manière préférée comprise entre 1 nm et 50 nm, en tenant compte du taux de dissolution et du temps nécessaire à la reconstruction de la paroi de vaisseau.

13. Endoprothèse vasculaire selon l'une quelconque des revendications précédentes, **caractérisée en ce que** l'on fait varier l'épaisseur de couche du revêtement (5.1) sur la surface des entretoises (2).
